(19)

**Europäisches Patentamt**
**European Patent Office**
**Office européen des brevets**

(11) **EP 4 069 118 B1**

(12) **EUROPEAN PATENT SPECIFICATION**

(45) Date of publication and mention
of the grant of the patent:
**19.07.2023 Bulletin 2023/29**

(21) Application number: **20819659.2**

(22) Date of filing: **30.11.2020**

(51) International Patent Classification (IPC):
**A61B 18/12** *(2006.01)* **H03K 3/335** *(2006.01)*

(52) Cooperative Patent Classification (CPC):
**A61B 18/1206; H03K 3/53;** A61B 2018/126;
A61B 2018/1286

(86) International application number:
**PCT/EP2020/083979**

(87) International publication number:
**WO 2021/110607 (10.06.2021 Gazette 2021/23)**

(54) **PULSE GENERATING CIRCUIT, AND ELECTROSURGICAL GENERATOR INCORPORATING THE SAME**

IMPULSGENERATORSCHALTUNG UND ELEKTROCHIRURGISCHER GENERATOR MIT DIESER SCHALTUNG

CIRCUIT DE GÉNÉRATION D'IMPULSIONS ET GÉNÉRATEUR ÉLECTROCHIRURGICAL RENFERMANT UN TEL CIRCUIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.12.2019 GB 201917693**

(43) Date of publication of application:
**12.10.2022 Bulletin 2022/41**

(73) Proprietor: **Creo Medical Limited**
**Chepstow, Monmouthshire NP16 5UH (GB)**

(72) Inventors:
• **HANCOCK, Christopher Paul**
**Chepstow Monmouthshire NP16 5UH (GB)**
• **DAVIES, Ilan**
**Chepstow Monmouthshire NP16 5UH (GB)**
• **HODGKINS, George**
**Chepstow Monmouthshire NP16 5UH (GB)**

(74) Representative: **Mewburn Ellis LLP**
**Aurora Building**
**Counterslip**
**Bristol BS1 6BX (GB)**

(56) References cited:
**US-A1- 2019 216 533**

• **Tampieri Daniele: "Avalanche transistor", , 30 November 2019 (2019-11-30), XP055772227, Wikipedia Retrieved from the Internet: URL:https://en.wikipedia.org/w/index.php?t itle=Avalanche_transistor&oldid=928674358 [retrieved on 2021-02-04]**
• **WERNER B HERDEN: "Application of avalanche transistors to circuits with a long mean time to failure", IEEE TRANSACTIONS ON INSTRUMENTATION AND MEASUREMENT, IEEE SERVICE CENTER, PISCATAWAY, NJ, US, vol. IM-25, no. 2, 1 June 1976 (1976-06-01), pages 152-160, XP011464300, ISSN: 0018-9456, DOI: 10.1109/TIM.1976.6312331**

## Description

TECHNICAL FIELD

The present invention relates to a pulse generating circuit for an electrosurgical generator and to an electrosurgical generator incorporationg said pulse generating circuit.

BACKGROUND TO THE INVENTION

Electrosurgical generators are pervasive throughout hospital operating theatres, for use in open and laparoscopic procedures, and are also increasingly present in endoscopy suites. In endoscopic procedures the electrosurgical accessory is typically inserted through a lumen inside an endoscope. Considered against the equivalent access channel for laparoscopic surgery, such a lumen is comparatively narrow in bore and greater in length.

WO 2019/185331 A1 discloses an electrosurgical generator capable of supplying energy in a waveform that causes electroporation in biological tissue. The electrosurgical generator may comprise an electroporation waveform supply unit that is integrated with means for generating microwave electromagnetic signals and radiofrequency electromagnetic signals for treatment. The electrosurgical generator may be configured to deliver different types of energy along a common feed cable. The electroporation waveform supply unit comprises a DC power supply and a DC pulse generator. The DC power supply may include a DC-DC converter for up-converting a voltage output by an adjustable voltage supply. Each DC pulse may have a duration in the range 1 ns to 10 ms and a maximum amplitude in the range 10 V to 10 kV.

Tampieri Daniele: "Avalanche transistor", published on Wikipedia on November 30th 2019, describes a pulse generating circuit for an electrosurgical generator comprising a voltage source connected to a load and an avalanche transistor as switching element, an open circuit coaxial transmission line connected between the switching element and the voltage source to be charged by the voltage source when the switching element is in an OFF state and to be discharged when the switching element is in an ON state and a trigger pulse generator to generate a trigger pulse to activate the avalanche transistor.

Werner B Herden: "Application of avalanche transistors to circuits with a long mean time to failure",IEEE Transactions on instrumentation and measurement, vol. IM-25, no. 2, 1 June 1976, pages 152-160, describes a pulse generating circuit with the switching element comprising a plurality of series connected avalanche transistors.

In recent years there have been numerous developments of ultrashort electric field pulse generators [1]. Ultrashort electric field pulses in the nanosecond regime have numerous applications. Applications includes: measurement of particles, photography, ultra-wideband radar detection and medical application to name a few [2]-[3].

There are numerous methods of generating high amplitude, nanosecond pulsed electric field with a rise and fall time of 2 ns. Traditionally, coaxial transmission line-based implementations, such as Blumlein, in correlation with spark-gap, Marx bank, or diode and laser opening switch techniques have been used to generate high-voltage nanosecond pulses [1].

SUMMARY OF THE INVENTION

The invention is defined by the appended independent claim. Preferred embodiments of the invention are illustrated in the dependent claims.

At its most general the present invention provides a pulse generating circuit for an electrosurgical generator, which is configured to generate high voltage pulses having a duration less than 10 ns suitable for causing electroporation of biological cells. In particular, the pulse generating circuit disclosed herein may be suitable for generating pulses that exhibit a 'flat-top' profile, i.e. having steep (e.g. less than 2 ns) rise and fall times, with minimal ringing. As explained in more detail below, this can be achieved through an open circuit transmission line technique in conjunction with the stacking of avalanche transistors as a fast switching element.

According to the invention there is provided a pulse generating circuit for an electrosurgical generator, the pulse generating circuit comprising: a voltage source connectable to a load via a switching element; an open circuit coaxial transmission line connected between the switching element and the voltage source to be charged by the voltage source when the switching element is in an OFF state and to be discharged when the switching element is in an ON state, wherein the switching element comprises: a plurality of series connected avalanche transistors; and a trigger pulse generator configured to generate a trigger pulse to activate the plurality of series connected avalanche transistors, and wherein the impedance of the coaxial transmission line is configured to match a sum of (i) the impedance the plurality of series connected avalanche transistors, and (ii) the impedance of the load. This circuit configuration can yield a flat top pulse (due to the matched impedance condition) having a short duration (controlled by the length of the coaxial transmission line) with a ultrashort rise time (controlled by the switching element) and an amplitude suitable for electroporation due to the cascading effect of the series connected avalanche transistors. In particular, the amplitude of the output may be 500 V or more, e.g. 1 kV or more, without exceeding the collector-base breakdown voltage across any of the plurality of series connected avalanche transistors.

The coaxial transmission line may have a length selected to provide a line delay equal to or less than 5 ns. The pulse duration is twice the line delay, so the output pulse may have duration equal to or less than 10 ns.

The coaxial transmission line may be charged by the voltage source through a resistor having a high impedance, e.g. 1 MΩ. The circuit may thus be considered as comprises a first loop when the switching element is in an OFF state and a second loop when the switching element is in an ON state. In the first loop, current flows from the voltage source through the resistor to charge the coaxial transmission line. In the second loop, current flows from the coaxial transmission line through the switching element to the load.

The trigger pulse may comprise a TTL signal. The trigger pulse generator may be any source suitable for generating such a signal, e.g. a microprocessor or the like. The trigger pulse may have a voltage less than the emitter-base breakdown voltage of each of the plurality of avalanche transistors. The duration of the trigger pulse may be longer than the duration of the pulse from the coaxial transmission line, to ensure that the switching element is in an ON state for long enough for the coaxial transmission line to completely discharge. In one example, the trigger pulse has a voltage of 5 V and a duration of 600 ns.

The trigger pulse generator may be connected to the plurality of series connected avalanche transistors via a transformer. This means that the trigger signal is floating between the base and emitter, and is therefore independent of the voltage through the transistor and on to the load. In one example, the trigger pulse may be applied between the collector and emitter of a first transistor of the plurality of series connected avalanche transistors. The first transistor may be the transistor that is furthest from the coaxial transmission line.

A diode may be connected in parallel with each of the plurality of series connected avalanche transistors to clamp the voltage across each transistor to less than its collector-base breakdown voltage. This protects the transistors.

Each transistor in the plurality of series connected avalanche transistors may be identical so that a voltage of the voltage source is divided evenly between the transistors.

As mentioned above, the present invention is particularly suited for use in electrosurgery. The load may therefore comprise an electrosurgical instrument capable of delivered a monopolar pulse for electroporation of biological tissue.

The invention provides an electrosurgical generator having a pulse generating circuit as set out above.

BRIEF DESCRIPTION OF THE DRAWINGS

Examples

are discussed below with reference to the accompanying drawings, in which:

Fig. 1 is a schematic diameter that illustrates the principle of a discharge line generator with an ideal switch;

Fig. 2 is a graph showing a voltage waveform at (i) the transmission line, and (ii) the load in Fig. 1;

Fig. 3A is a schematic diagram representing the open circuit transmission line of Fig. 1 in a DC model;

Fig. 3B is a schematic diagram representing the open circuit transmission line of Fig. 1 in a transmission line model;

Fig. 4 is a schematic diagram of showing the open circuit transmission line of Fig. 1 with an avalanche transistor to generate an ultrashort electric field pulse;

Fig. 5 is a diagram of a simulated LTSpice circuit of a monopolar ultrashort electric field pulse generator;

Fig. 6 is a graph showing pulses of various durations generated from the LTSpice circuit of Fig. 5; and

Fig. 7 is a monopolar pulse observed with a matched 35 Q load, from circuit in Fig. 5.

DETAILED DESCRIPTION, FURTHER OPTIONS AND PREFERENCES

Generation of ultra-short pulses is possible by using an open circuit coaxial transmission line as a high-Q storage element consisting of distributed series of inductors and shunt capacitors with minimal resistance and shunt conductance. Discharging an open ended delay line via a fast switching element provides a means of producing a 'flat-top' rectangular pulse with steep fall times of less than 2 ns in a simple and affordable manner. The co-axial transmission line with a characteristic impedance $Z_0$ a length of $l$ and a dielectric constant $\varepsilon_r$ is charged to a voltage level $V_{cc}$ through a high impedance resistor $R_c$. The line will have and associated delay time $T$ given by the following equation:

$$T = \frac{l\sqrt{\varepsilon_r}}{c}$$

where $c$ is the speed of light ($2.99 \times 10^8$ m/s).

It follows from this that the pulse duration associated with the transmission line is:

$$2T = 2\frac{l\sqrt{\varepsilon_r}}{c}$$

An ultrashort electric field pulse can be generated on a load, $R_L$, by discharging the transmission line through $R_L$ by closing a switching element. The switching element determines the rise time of the ultrashort electric field pulse whilst the transmission line determines the pulse duration (or width) and the fall time.

As explained above, the duration of the pulse at the load will be twice the associated delay time of the transmission line.

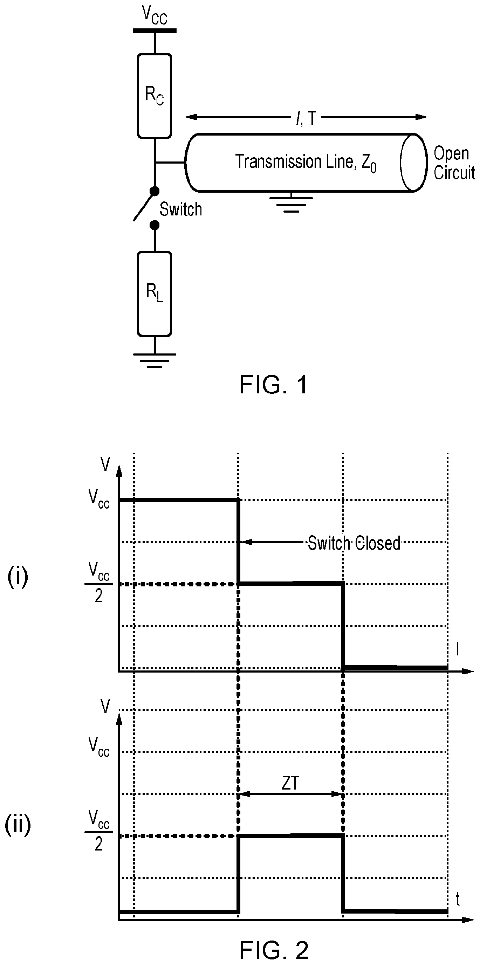

Fig. 1 illustrates the principle of an open circuit transmission line technique with an ideal switch as the switching element.

Fig. 2 shows the voltage waveforms obtained from the system of Fig. 1 at (i) the transmission line $Z_0$ and (ii) load $RL.$

The relationship between the characteristic impedance of the transmission line $Z_0$ and the load $R_L$ is integral to the performance of an open circuit coaxial transmission line technique in two ways, which can be understood by modelling the configuration using direct circuit (DC) theory and transmission line theory.

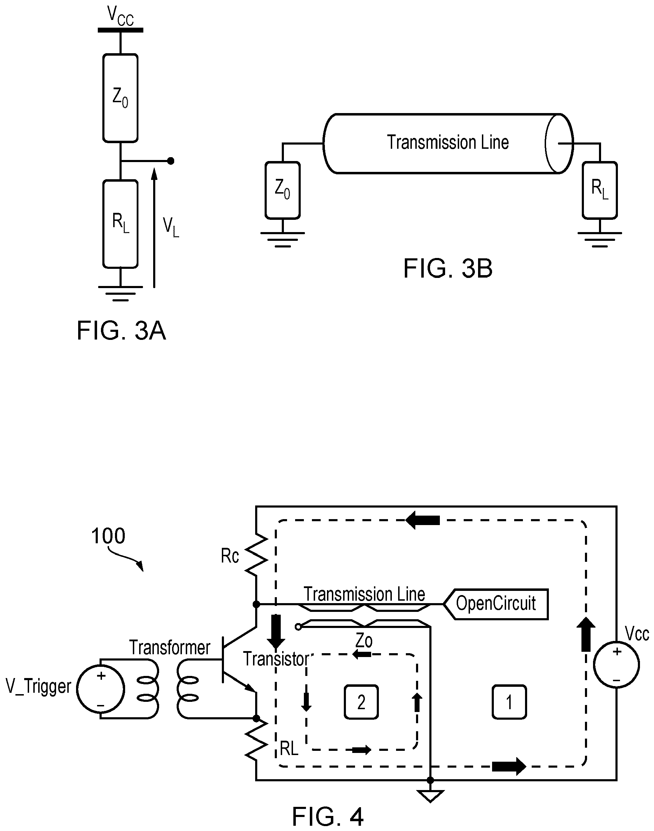

In DC theory, the relationship between $Z_0$ and $R_L$ imitates a potential divider, as shown in Fig. 3A. Their relationship determines the pulse amplitude at the load $V_L$:

$$V_L = \left(\frac{R_L}{R_L + Z_0}\right)V_{cc}$$

If the impedance $Z_0$ is the same as $R_L$, the maximum amplitude of the pulse at the load, $V_{Lmax}$, will be half the voltage the to which the transmission line is charged:

$$if\ R_L = Z_0,\quad V_{Lmax} = \frac{V_{cc}}{2}$$

Using a transmission line model, the system can be represented as shown in Fig. 3B. In this model, the relationship between $Z_0$ and $R_L$ determines the reflection coefficient, and therefore the pulse shape at the load. If $R_L$ is the same as $Z_0$, the reflection coefficient will be zero and no secondary pulse or reflection of the primary pulse will be seen at the load:

$$\Gamma = \left(\frac{R_L - Z_0}{R_L + Z_0}\right) \qquad \therefore if\ R_L = Z_0,\ \ \Gamma = 0$$

Thus, the relationship of $Z_0$ and $R_L$ determine two key aspects of the pulse at a load: (i) the pulse amplitude, and (ii) pulse shape (caused by any reflection). It follows from the analysis above, that the best pulse shape and parameters, the characteristic impedance of the transmission line $Z_0$ and the load $R_L$ should match.

Other features of the pulse are controlled by other parameters of the circuit. For example, the pulse risetime is determined by the behavioural of the switching element, whilst the pulse width is determined by the length of the transmission line, as discussed above.

This switching element in embodiments of the invention is preferably provided by a stacked array of avalanche transistors. An avalanche transistor is known to provide reliable and repeatable high-speed switching of high voltages with rise times as low as 300 ps, which can be achieved in practice if microwave component layout techniques are considered when the circuit are implemented. Avalanche transistors utilize the negative-resistance characteristics region of bipolar junction transistors, which result from operation in the common-emitter breakdown region. The avalanche region lies between collector emitter ($V_{CEO}$) and collector base ($V_{CBO}$) voltage when the base current $I_B$ = 0 A and emitter current $I_E$ = 0 A.

Fig. 4 is a schematic diagram of a pulse generating circuit 100 that utilises an open circuit transmission line technique in combination with an avalanche transistor as a fast switching element. The circuit function is based on the discharge of the open-circuit transmission line across an avalanche transistor into a load $R_L$.

A single avalanche transistor circuit can be configured to have a bi-stable operation, where the maximum pulse amplitude at the output is limited to half the value of the transistor's collector-emitter breakdown voltage, $BV_{CES}$, if $Z_0 = R_L$. A supply voltage $V_{cc}$ above the transistor's $BV_{CES}$ would permanently breakdown and damage the avalanche transistors as a switching element.

Initially, energy is stored in a co-axial transmission line via a small current flow in loop 1. A positive trigger on the base of the transistor will suddenly switch the transistor 'on'. The energy stored in the transmission line will simultaneously be released as a high current along loop 2, producing a pulse on $R_L$. The width of the trigger on the base is longer than $2T$, i.e. the required pulse width at the load.

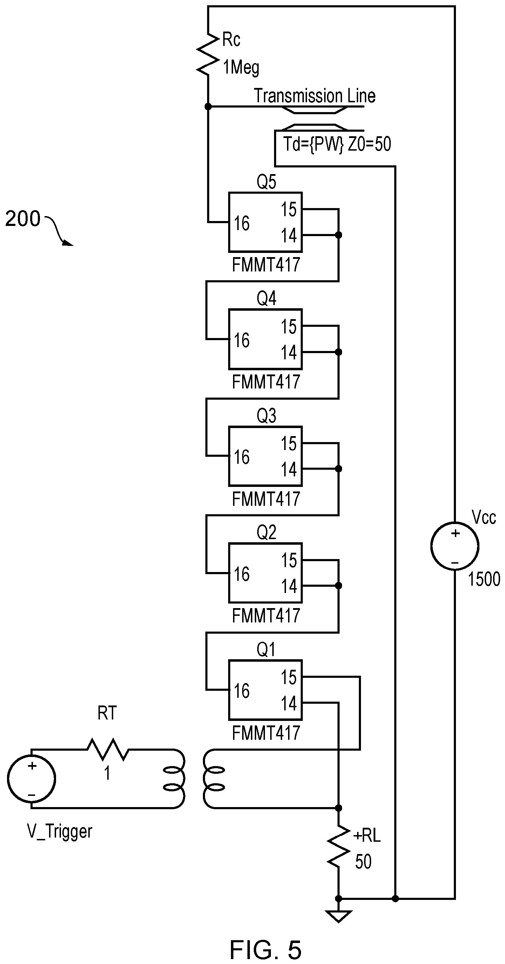

Fig. 5 shows a pulse generation circuit 200 that is an embodiment of the invention. The pulse generation circuit 200 is similar to the circuit shown in Fig. 4, except that in place of the single avalanche transistor, and according to the invention, there is a plurality (five in this example) of series-connected avalanche transistors. The plurality of series-connected avalanche transistors effectively operate in combination as a single avalanche transistor. This means that the discharge of the open-circuit transmission line is across the stacked transistors to the load, thereby resulting in a cascade effect that causes a proportionally higher pulse amplitude at the load. In this example, each of the avalanche transistors is identical so that the supply voltage $V_{cc}$ is equally distributed across each of the avalanche transistor in the series chain.

In this arrangement, the maximum pulse amplitude that can be generated is dependent on the number of stacked avalanche transistor $n$. The number of avalanche transistors required to generate a specific pulse amplitude $V_L$ can be expressed as

$$V_L = nBV_{CBO}\left(\frac{R_L}{R_L + Z_0}\right)$$

where $BV_{CBO}$ is the collector-base breakdown voltage of each avalanche transistor. If $R_L = Z_0$, a maximum pulse amplitude $V_{Lmax}$ can thus be expressed as

$$V_{Lmax} = \frac{nBV_{CBO}}{2}$$

In the pulse generating circuit 200 five FMMT417 avalanche transistor are stacked. Each transistor has an collector-emitter breakdown voltage $BV_{CEO}$ of 100 V and a collector-base breakdown voltage $BV_{CBO}$ of 320 V. The circuit shown in Fig. 5 was simulated using LTSpice models. The Spice model of the FMMT417 was directly taken from the manufacture's website. The source resistance $R_c$ is 1 MΩ, characteristic impedance of the transmission line $Z_0$ is 50 Ω, source voltage $V_{cc}$ is 1.5kV.

The circuit may include a diode (not shown) connected in parallel with each transistor to clamp the voltage to ensure that the voltage across each transistor does not exceed its collector-base breakdown voltage. Doing so can increase the lifespan of the transistors and ensure that triggering occurs by the trigger signal.

The trigger signal may be provided by any suitable source. Preferably the trigger signal is generated by a TTL source or a microcontroller. In this example, the trigger signal comprises a pulse having a duration of 600 ns and a 5 V amplitude and pulse period (period of repetition) of 20 ms. It is advantageous to have a 5 V trigger signal because it is less than the emitter-base breakdown voltage of the transistors.

The pulse width of trigger signal is arranged to be longer than the pulse desired to be generated from the transmission line. The duration of 600 ns was chosen in this case to provide a safe margin to allow the whole transmission line to discharge.

The trigger signal repetition rate (pulse period) is limited by the time it takes for the open-circuit charged transmission line to charge up again to full capacity.

A transformer is disposed between the trigger signal generator and the base and emitter of the first transistor in the stack (i.e. the transistor furthest from the transmission line). This configuration means that the trigger pulse is floating, and therefore should be the same between the base and emitter of the first transistor no matter the voltage through the transistor and onto the load. As a result, the amplitude of the pulse at the load ought to increase linearly with the number of transistors in the stack. The transformer may be a 1-EMR-046 Gate Drive Transformer having a 1:1 winding ratio and high voltage isolation.

In use, the five stacked avalanche transistors are initially in their off-state, with each transistor having 300 V across them (i.e. $V_{cc}/n$). When a positive trigger signal is applied to the base of the first transistor Q1, Q1 is turned 'on' and places its collector voltage near ground potential. This results in the second transistor Q2 having twice the collector-emitter voltage, thus creating the desired condition in terms of overvolting and therefore causes a non-destructive avalanching of Q2 and places its collector near ground potential. This creates a sequential 'knock-on' effect on the next transistor in the chain resulting in the overvolting of the first avalanche transistors, Q1, to the final avalanche transistors, Q5 near the charged open circuit transmission line. When Q5 is turned 'on', a fast rise time is produced at the load (< 2 ns), therefore allowing the charged open circuit transmission line to discharge through the load producing a pulse with a width of $2T$ and a maximum amplitude of $V_{cc}/2$, if $R_L = Z_0$.

The pulse generating circuit 200 may thus be used to generate monopolar ultrashort electric field pulses.

Although not shown in Fig. 5, the pulse generating circuit 200 may further comprise a capacitor connected in parallel with the high impedance resistor $R_c$. This acts as a current reservoir to enable high current to be provided for a short period of time without causes a significant voltage drop. For example, if it is desired to produce a pulse having a 1 kV peak voltage, the driving current $I_d$ required can be calculated as $I_d = C\frac{dV}{dt}$, where $C$ is the load capacitance (i.e. the combined capacitance of the coaxial transmission line and $R_L$), and $\frac{dV}{dt}$ is the desired change in voltage over the pulse rise time. The driving current $I_d$ may be 1500 A or more, e.g. 2000 A. The reservoir capacitor prevents the voltage of the pulse from dropping significantly through the duration of the pulse. The capacitance value $C_{res}$ of the reservoir capacitor can be calculated by considering an acceptable drop in voltage $\frac{dV}{dt}$ (say 1 V over a 10 ns pulse) for the driving current $I_d$. The capacitance value $C_{res}$ may be 1.5 to 2.5 μF, for example.

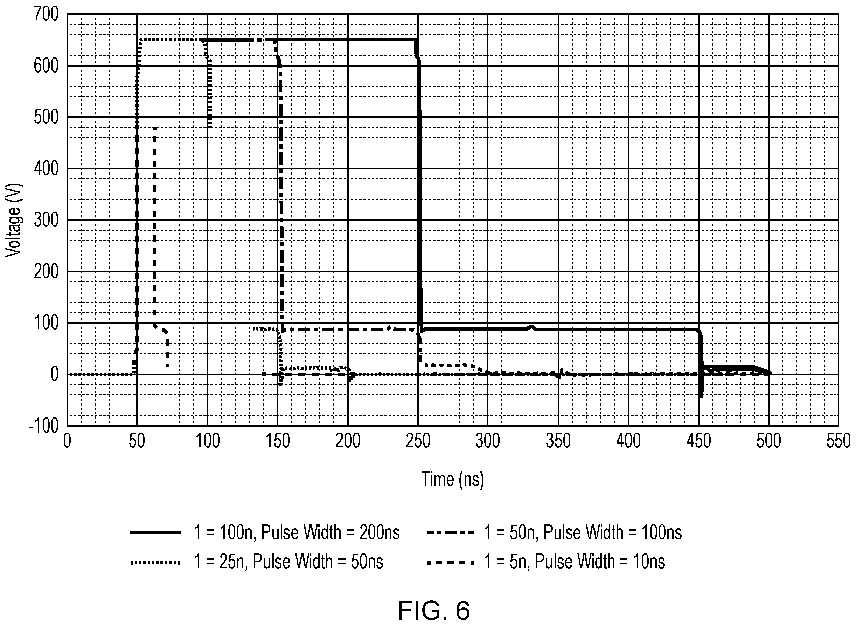

Fig. 6 is a graph showing voltage pulses obtained for a range of transmission line lengths. In Fig. 6, the transmission line lengths are characterised by the line delay $T$. The graph demonstrate that the transmission line length determines the pulse width of $2T$, i.e. transmission lines having line delays of 5 ns, 25 ns, 50 ns and 100 ns produce pulse widths of 10 ns, 50 ns, 100 ns and 200 ns respectively. Additionally, the rise times of all four pulses are the same and less than 2ns, which emphasises that the switching element, i.e. the five avalanche transistors, determines this factor.

The graph in Fig. 6 suggests that a 50 Ω load does not match the transmission line characteristic impedance because secondary pulse of lower amplitude to the primary pulse is seen on each signal. This suggested an unmatched load due to reflection, i.e. $\Gamma \neq 0$. According to the invention, it is necessary to compensate for the impedance of the transistors in order to optimise the pulse generation circuit. In the example shown in Fig. 5, each individual transistor has an impedance of ~3 Q. Therefore, a total of ~15 S2 is across the transistor stack. The reflection coefficient can thus be expressed as

$$\Gamma = \left(\frac{R_\Sigma - Z_0}{R_\Sigma + Z_0}\right) = \left(\frac{(R_L + nR_A) - Z_0}{(R_L + nR_A) + Z_0}\right)$$

wherein the $R_\Sigma$ is the total impedance of the circuit, and $R_A$ is the impedance of a signal avalanche transistor.

This explains the reflection observed in the pulses shown in Fig. 6, as $\Gamma$ = 0.13, and the amplitude of the reflection pulse is ~13% of the primary pulse ($R_L$ = 50 $\Omega$, $nR_A$ = (3 $\Omega \times$ 5) = 15 $\Omega$ and $Z_0$ = 50 $\Omega$). The additional impedance of $nR_A$ also affects the DC component of the design, which can be rewritten as :

$$V_L = \left(\frac{R_L}{Z_0 + R_A + R_L}\right) V_{cc}$$

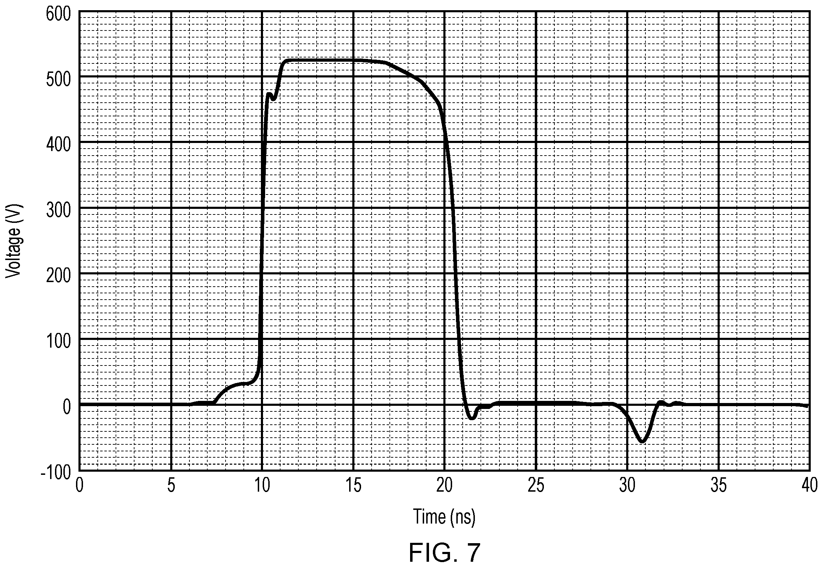

Taking this into account, the impedance of the load $R_L$ was adjusted to 35 Q. This resulted in a single monopolar pulse at the load with zero reflection and no secondary pulse, as shown in Fig. 7.

REFERENCES

[1] W. Meiling and F. Stary, Nanosecond pulse techniques. New York: Gordon and Breach, 1970, p. 304.
[2] Q. Yang, X. Zhou, Q.-g. Wang and M. Zhao, "Comparative analysis on the fast rising edge pulse source with two kinds of avalanche transistor," in Cross Strait Quad-Regional Radio Science and Wireless Technology Conference, Chengdu, 2013.
[3] G. Yong-sheng et al., "High-speed, high-voltage pulse generation using avalanche transistor," Review of Scientific Instruments, vol. 87, no. 5, p. 054708, 2016.

**Claims**

1. A pulse generating circuit for an electrosurgical generator, the pulse generating circuit comprising:

   a voltage source connectable to a load via a switching element;
   an open circuit coaxial transmission line connected between the switching element and the voltage source to be charged by the voltage source when the switching element is in an OFF state and to be discharged when the switching element is in an ON state,
   wherein the switching element comprises:

   a plurality of series connected avalanche transistors; and
   a trigger pulse generator configured to generate a trigger pulse to activate the plurality of series connected avalanche transistors, and

   wherein the impedance of the coaxial transmission line is configured to match a sum of (i) the impedance the plurality of series connected avalanche transistors, and (ii) the impedance of the load.

2. A pulse generating circuit according to claim 1, wherein the coaxial transmission line has a length selected to provide a line delay equal to or less than 5 ns.

3. A pulse generating circuit according to claim 1 or 2, wherein the coaxial transmission line is charged by the voltage source through a resistor.

4. A pulse generating circuit according to any preceding claim, wherein the trigger pulse generator comprises a TTL device.

5. A pulse generating circuit according to any preceding claim, wherein the trigger pulse has a voltage less than the emitter-base breakdown voltage of each of the plurality of avalanche transistors.

6. A pulse generating circuit according to any preceding claim, wherein the trigger pulse generator is connected to the plurality of series connected avalanche transistors via a transformer.

7. A pulse generating circuit according to any preceding claim, wherein the trigger pulse is applied between the collector and emitter of a first transistor of the plurality of series connected avalanche transistors.

8. A pulse generating circuit according to claim 7, wherein the first transistor is furthest from the coaxial transmission line.

9. A pulse generating circuit according to any preceding claim, wherein a diode is connected in parallel with each of the plurality of series connected avalanche transistors to clamp the voltage across each transistor to less than its collector-base breakdown voltage.

10. A pulse generating circuit according to any preceding claim, wherein each transistor in the plurality of series connected avalanche transistors is identical.

11. A pulse generating circuit according to any preceding claim, wherein the load is an electrosurgical instrument.

**12.** An electrosurgical generator having a pulse generating circuit according to any preceding claim.

## Patentansprüche

**1.** Impulserzeugungsschaltung für einen elektrochirurgischen Generator, wobei die Impulserzeugungsschaltung Folgendes umfasst:

eine Spannungsquelle, die über ein Schaltelement mit einer Last verbindbar ist;
eine Leerlauf-Koaxialübertragungsleitung, die zwischen dem Schaltelement und der Spannungsquelle verbunden ist, um durch die Spannungsquelle geladen zu werden, wenn sich das Schaltelement in einem AUS-Zustand befindet, und entladen zu werden, wenn sich das Schaltelement in einem EIN-Zustand befindet, wobei das Schaltelement Folgendes umfasst:

eine Vielzahl von in Reihe geschalteten Avalanche-Transistoren; und
einen Auslöseimpulsgenerator, der ausgelegt ist, um einen Auslöseimpuls zu erzeugen, um die Vielzahl der in Reihe geschalteten Avalanche-Transistoren zu aktivieren, und
wobei die Impedanz der Koaxialübertragungsleitung ausgelegt ist, um an eine Summe aus (i) der Impedanz der Vielzahl von in Reihe geschalteten Avalanche-Transistoren und (ii) der Impedanz der Last angepasst zu sein.

**2.** Impulserzeugungsschaltung nach Anspruch 1, wobei die Koaxialübertragungsleitung eine Länge aufweist, die ausgewählt ist, um eine Leitungsverzögerung kleiner oder gleich 5 ns bereitzustellen.

**3.** Impulserzeugungsschaltung nach Anspruch 1 oder 2, wobei die Koaxialübertragungsleitung durch einen Widerstand von der Spannungsquelle geladen wird.

**4.** Impulserzeugungsschaltung nach einem der vorangegangenen Ansprüche, wobei der Auslöseimpulsgenerator eine TTL-Vorrichtung umfasst.

**5.** Impulserzeugungsschaltung nach einem der vorangegangenen Ansprüche, wobei der Auslöseimpuls eine Spannung aufweist, die kleiner ist als die Durchbruchspannung der Emitter-Basis jedes aus der Vielzahl von Avalanche-Transistoren.

**6.** Impulserzeugungsschaltung nach einem der vorangegangenen Ansprüche, wobei der Auslöseimpulsgenerator mit der Vielzahl der in Reihe geschalteten Avalanche-Transistoren über einen Transformator verbunden ist.

**7.** Impulserzeugungsschaltung nach einem der vorangegangenen Ansprüche, wobei der Auslöseimpuls zwischen dem Kollektor und dem Emitter eines ersten Transistors aus der Vielzahl von in Reihe geschalteten Avalanche-Transistoren angelegt wird.

**8.** Impulserzeugungsschaltung nach Anspruch 7, wobei der erste Transistor am weitesten von der Koaxialübertragungsleitung weg angeordnet ist.

**9.** Impulserzeugungsschaltung nach einem der vorangegangenen Ansprüche, wobei eine Diode parallel mit jedem aus der Vielzahl von in Reihe geschalteten Avalanche-Transistoren geschaltet ist, um die Spannung an jedem Transistor auf weniger als dessen Kollektor-Basis-Durchbruchspannung zu klemmen.

**10.** Impulserzeugungsschaltung nach einem der vorangegangenen Ansprüche, wobei jeder Transistor aus der Vielzahl von in Reihe geschalteten Avalanche-Transistoren identisch ist.

**11.** Impulserzeugungsschaltung nach einem der vorangegangenen Ansprüche, wobei die Last ein elektrochirurgisches Instrument ist.

**12.** Elektrochirurgischer Generator, der eine Impulserzeugungseinheit nach einem der vorangegangenen Ansprüche aufweist.

## Revendications

**1.** Circuit de génération d'impulsion pour un générateur électrochirurgical, le circuit de génération d'impulsion comprenant :

une source de tension pouvant être connectée à une charge via un élément de commutation ;
une ligne de transmission coaxiale de circuit ouvert connectée entre l'élément de commutation et la source de tension, destinée à être chargée par la source de tension lorsque l'élément de commutation est dans un état éteint et à être déchargée lorsque l'élément de commutation est dans un état allumé,
dans lequel l'élément de commutation comprend :

une pluralité de transistors à avalanche connectés en série ; et
un générateur d'impulsion de déclenchement configuré pour générer une impulsion de déclenchement pour activer la pluralité de transistors à avalanche connectés en série, et

dans lequel l'impédance de la ligne de transmission coaxiale est configurée pour correspondre à une somme (i) de l'impédance de la pluralité de transistors à avalanche connectés en série, et (ii) de l'impédance de la charge.

2. Circuit de génération d'impulsion selon la revendication 1, dans lequel la ligne de transmission coaxiale présente une longueur choisie pour fournir un retard de ligne égal ou inférieur à 5 ns.

3. Circuit de génération d'impulsion selon la revendication 1 ou 2, dans lequel la ligne de transmission coaxiale est chargée par la source de tension à travers une résistance.

4. Circuit de génération d'impulsion selon l'une quelconque des revendications précédentes, dans lequel le générateur d'impulsion de déclenchement comprend un dispositif TTL.

5. Circuit de génération d'impulsion selon l'une quelconque des revendications précédentes, dans lequel l'impulsion de déclenchement présente une tension inférieure à la tension de claquage de base-émetteur de chacun de la pluralité de transistors à avalanche.

6. Circuit de génération d'impulsion selon l'une quelconque des revendications précédentes, dans lequel le générateur d'impulsion de déclenchement est connecté à la pluralité de transistors à avalanche connectés en série via un transformateur.

7. Circuit de génération d'impulsion selon l'une quelconque des revendications précédentes, dans lequel l'impulsion de déclenchement est appliquée entre le collecteur et l'émetteur d'un premier transistor de la pluralité de transistors à avalanche connectés en série.

8. Circuit de génération d'impulsion selon la revendication 7, dans lequel le premier transistor est le plus éloigné de la ligne de transmission coaxiale.

9. Circuit de génération d'impulsion selon l'une quelconque des revendications précédentes, dans lequel une diode est connectée en parallèle avec chacun de la pluralité de transistors à avalanche connectés en série pour fixer la tension aux bornes de chaque transistor à une valeur inférieure à sa tension de claquage de base-collecteur.

10. Circuit de génération d'impulsion selon l'une quelconque des revendications précédentes, dans lequel chaque transistor de la pluralité de transistors à avalanche connectés en série est identique.

11. Circuit de génération d'impulsion selon l'une quelconque des revendications précédentes, dans lequel la charge est un instrument électrochirurgical.

12. Générateur électrochirurgical présentant un circuit de génération d'impulsion selon l'une quelconque des revendications précédentes.

FIG. 1

FIG. 2

FIG. 3A

FIG. 3B

FIG. 4

FIG. 5

FIG. 6

EP 4 069 118 B1

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019185331 A1

**Non-patent literature cited in the description**

- **TAMPIERI DANIELE.** Avalanche transistor. *Wikipedia,* 30 November 2019
- **WERNER B HERDEN.** Application of avalanche transistors to circuits with a long mean time to failure. *IEEE Transactions on instrumentation and measurement,* 01 June 1976, vol. IM-25 (2), 152-160
- **W. MEILING ; F. STARY.** Nanosecond pulse techniques. Gordon and Breach, 1970, 304
- **Q. YANG ; X. ZHOU ; Q.-G. WANG ; M. ZHAO.** Comparative analysis on the fast rising edge pulse source with two kinds of avalanche transistor. *Cross Strait Quad-Regional Radio Science and Wireless Technology Conference, Chengdu,* 2013
- **G. YONG-SHENG et al.** High-speed, high-voltage pulse generation using avalanche transistor. *Review of Scientific Instruments,* 2016, vol. 87 (5), 054708